Europäisches Patentamt

European Patent Office

Office européen des brevets

(19)

(11) Publication number: **0 157 844**
**B1**

## (12) EUROPEAN PATENT SPECIFICATION

(45) Date of publication of patent specification: **24.08.88**

(51) Int. Cl.⁴: **A 61 F 5/44**

(21) Application number: **84903646.2**

(22) Date of filing: **27.09.84**

(86) International application number:
**PCT/GB84/00331**

(87) International publication number:
**WO 85/01438 11.04.85 Gazette 85/09**

(54) INCONTINENCE DEVICES FOR WOMEN.

(30) Priority: 27.09.83 GB 8325847
27.09.83 GB 8325848
18.09.84 GB 8423591

(43) Date of publication of application:
16.10.85 Bulletin 85/42

(45) Publication of the grant of the patent:
24.08.88 Bulletin 88/34

(84) Designated Contracting States:
AT BE CH DE FR LI LU NL SE

(56) References cited:
US-A-2 638 093
US-A-3 116 734

(73) Proprietor: EAKIN, Thomas George
965 Upper Newtownards Road Dundonald
Belfast BT16 ORL (GB)

(72) Inventor: EAKIN, Thomas George
965 Upper Newtownards Road Dundonald
Belfast BT16 ORL (GB)

(74) Representative: Sheard, Andrew Gregory et al
Kilburn & Strode 30, John Street
London WC1N 2DD (GB)

Courier Press, Leamington Spa, England.

## Description

The present invention relates to an incontinence device particularly adapted for the use of female patients.

The problem of incontinence in women has long been difficult to solve. In hospitals the use of a self-retaining catheter inserted into the urethra is normal practice, and while this successfully controls involuntary flow it brings with it the problem of infection. In addition, professional help is normally required in replacing the catheter, which makes it inconvenient for use when the patient is not in hospital. The use of known incontinence clothing has little more than an external cosmetic effect, and since urine flow is not prevented the patient remains continually wet and uncomfortable.

Incontinence in females may be a transient condition or a long term condition or an involuntary condition.

The present invention has a number of aspects which address these different conditions but all the aspects have in common the characteristic of an internal portion which is held in or urged into functional cooperation with the urethra by securement of an external portion to tensioning means secured to, or mounted on the users body externally or the users clothing.

The condition referred to above as transient may be when the incontinence has been caused by trauma such as an operation and the patient can be expected gradually to regain control of bladder function. Here a drainage version of the device in accordance with the invention may be most appropriate, since it will accommodate inadvertent bladder leakage but enable the user to observe her increasing degree of control and gain confidence therefrom. This form of device is also useful where the patient has long term incontinence and for example is bed ridden.

The condition referred to as long term may on the other hand require more complete control as indeed may the transient condition when the patient has left hospital. Here a restrictive version of the invention is used in which the internal portion of the device occludes the urethra so that the device is removed to discharge the bladder.

Hybrids of these two versions are also envisaged in which a drainage channel is provided in the device, but this is occludable either by operation of an external control device or has an occlusion which can be overcome internally by the user exerting muscular force to express urine from the bladder past the occlusion. The former arrangement is preferred.

The condition referred to above as involuntary is a further condition of female incontinence which is known as stress incontinence. This is a condition in which involuntary discharge of urine occurs only in certain circumstances such as when the person coughs or jumps. This is caused by the bladder dropping. Known techniques which have been applied to try to control this condition comprise the so-called watch spring pessary and a surgical procedure in which a ligature is placed round the urethra. Both techniques aim to relocate the urethra in the normal position. The former technique is not very effective, the device tending to slip from the correct position and the latter technique involves a surgical procedure.

The third version of the present invention addresses the problem of stress incontinence. Here the internal portion of the device in accordance with the present invention functions to relocate the urethra but is urged into the correct position by the external securement and tensioning of the device.

A known urine drainage device, designed to drain urine into a container, is described in US-A-3116734. This document sets out the features of the pre-characterising portion of claim 1.

The present invention, which encompasses both drainage devices of the general type described in US-A-3116734 as well as urethral occlusion devices, is characterised in that the external portion consists of a lower external limb which is generally shaped such that, in use, the tensioning means pulls the lower limb forwards so that the lower limb is positioned at least partially within the vulva and against the pubic bone.

Moreover, the present invention consists preferably of a simple moulded device which can be worn in the vagina, and which is easy to use in that it can easily be inserted and removed by the patient herself and worn, when required, at home. It may conveniently be moulded in a one-piece form which is cheap to manufacture and easy to clean or cheap enough to be disposable.

Duct means may be provided to permit discharge of urine through the device. These may extend from the opening of the urethra to outside the vaginal opening. In this form, the invention retains one of the advantages of the catheter in that the urine can still be observed and its volume measured for diagnostic purposes.

Alternatively, the internal limb may have occlusion means arranged in use to occlude the urethra and thus prevent discharge of urine from the bladder.

In one form of this version of the invention the protuberance is compressible and is such that it will prevent involuntary discharge of urine but permit voluntary discharge brought about by muscular activity of the user.

In another form of this aspect of the invention the protuberance can be increased in size or reduced in size by control means located externally in use.

The protuberance may comprise a fluid filled bag, e.g. a liquid or gas, e.g. air, filled bag, connected to pump means which in use are located externally.

Preferably, the occlusion means are, provided on the upper limb by a forwardly facing protuberance, the pressure of which on the vaginal wall causes or assists the urethral occlusion.

The device may also be held in position by a rearwardly-extending portion which is pressed against the dorsal vaginal wall.

This rearwardly-extending portion may be either a rigid loop attached to the upper limb, or a moulded extension to the base of the U, or preferably may be generally shaped so as to conform to the lower vaginal wall proximate to the vaginal opening. In this case, the device is preferably provided with a second duct to allow egress of natural vaginal fluids.

In another form of the invention a rearwardly extending portion extends from the region of the upper end of the interior portion and across the vagina laterally and extends into the region of the cervix or past it but stops short of the dorsall wall of the vagina. This assists in maintaining the device in the laterally correct position and is particularly useful in the stress incontinence version of the invention.

The device may be made of a rigid or semi-rigid substance and is preferably provided with soft pads or coverings for comfort.

The tensioning means may conveniently comprise a strap or cord attached at one end to a belt, the tension being used to the elasticity of the cord or that of the belt, or both, the belt also constituting the securing means.

It may be desirable, for comfort, for the strap to be of a soft or padded material or for it to be sheathed.

In another form of the invention the device is generally rigid but is provided with a flexible portion between the limbs allowing hinging motion therebetween.

In order to produce or assist in producing a fluid-tight seal between the anterior vaginal wall and the inner face of the first limb, a suitable substance of putty-like consistency may be used.

The first limb may be shorter than the second limb, or they may be of essentially equal length, but this is not essential; the upper limb may be significantly shorter provided the necessary seal can be maintained. On the other hand, an arrangement in which the lower or outer limit is shorter is not excluded.

The invention may be put into practice in various ways, and a number of specific embodiments will be described by way of example with reference to the accompanying drawings, in which:

Figure 1A is a perspective view of a first embodiment of the invention showing a drainage version of the invention;

Figure 1B is a front elevation of the device shown in Figure 1A;

Figure 2 is a medial cross section of the embodiment of Figures 1A and 1B illustrating the drainage channel and tube outlet;

Figure 3A is a medial cross section of the lower part of the female body, showing the embodiment of Figure 1 in position, in which position it is held either by an external strap (chained line) or by the external strap and an internal loop (dashed line);

Figure 3B is a view similar to Figure 3A showing a first modification of the first embodiment, provided with sheathing of the strap and a modified internal positioning arrangement.

Figure 4 illustrates one method of securing the embodiments of the invention in position, in which an extensible strap and belt are used;

Figure 5 is a perspective view of a second modification of the first embodiment of the invention, in which the device is shaped to conform to the vaginal opening as well as being held in place by a strap and belt;

Figure 6A is a medial cross section of this second modification of the first embodiment, illustrating the drainage channel and tube and showing a second conduit which allows egress of the natural vaginal fluids;

Figure 6B is a transverse cross-section of the arrangement shown in Figure 5 taken on the line VIB-VIB.

Figure 7 is a perspective view of the second embodiment of the invention showing a restrictive version of the invention;

Figure 8 is a medial cross section of the lower part of the female body showing the embodiment of Figure 7 in position, in which position it is held either by an external strap (see Figure 4) (chained line) or in a first modification of this second embodiment by the external strap and by an internal loop (dashed line);

Figure 9 is a perspective view of a second modification of this second embodiment of the invention, in which the device is shaped to conform to the vaginal opening as well as being held in place by a strap and belt (as in Figure 4);

Figure 10 is a medial cross section of the second modification of the second embodiment (shown in Figure 9) illustrating a conduit which allows egress of the natural vaginal fluid;

Figure 11 is a plan view from the front of a third modification of the second embodiment;

Figure 12 is a medial cross section of this third modification of the second embodiment;

Figure 13 is a plan view from the rear of the embodiment shown in Figures 11 and 12;

Figure 14 is a cross section similar to Figure 8 showing the third modification of the second embodiment in use;

Figure 15 is a perspective view from one side and behind of a fourth modification of the second embodiment of the invention in which an overcomable occlusion means is provided, the general shape of the device being similar to the third modification of the second embodiment;

Figure 16 is a cross section on the line XVI-XVI of Figure 15;

Figure 17 is a side elevation of a fifth modification of the second embodiment of the invention in which a controllable occlusion means is provided, the device otherwise being similar to that shown in Figure 15;

Figure 18 is a cross section on the line XVIII-XVIII of Figure 17 showing in full lines the open position of the occlusion means and in dotted lines the closed position;

Figure 19 is a perspective view from one side and behind of a first form of the third embodiment, the stress incontinence version, of the invention; and

Figure 20 is a similar view to Figure 19 of a second form or first modification of the third embodiment of the invention which is provided with a dorsal extension similar to that shown in Figure 3B.

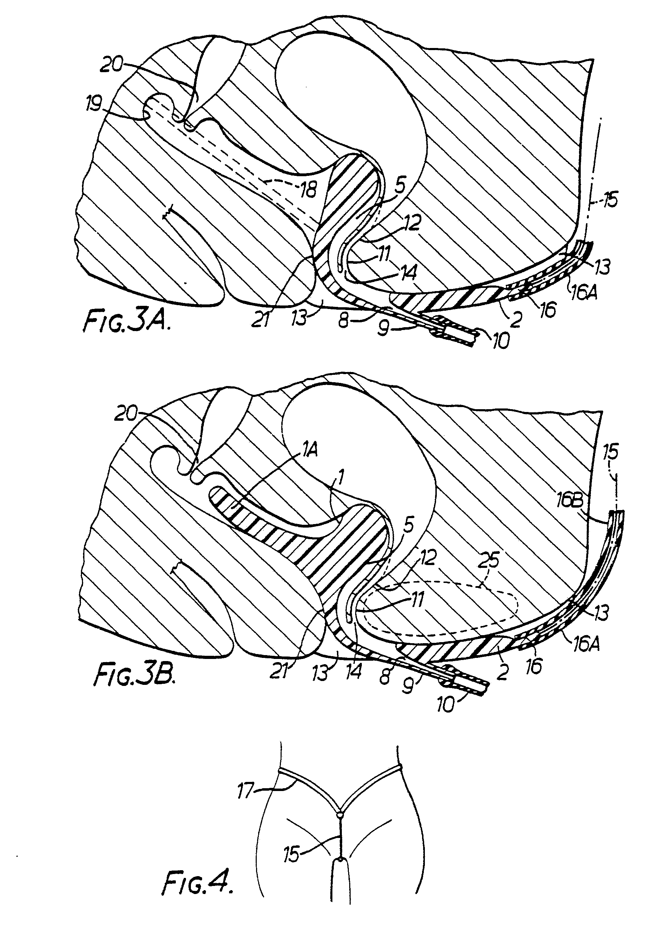

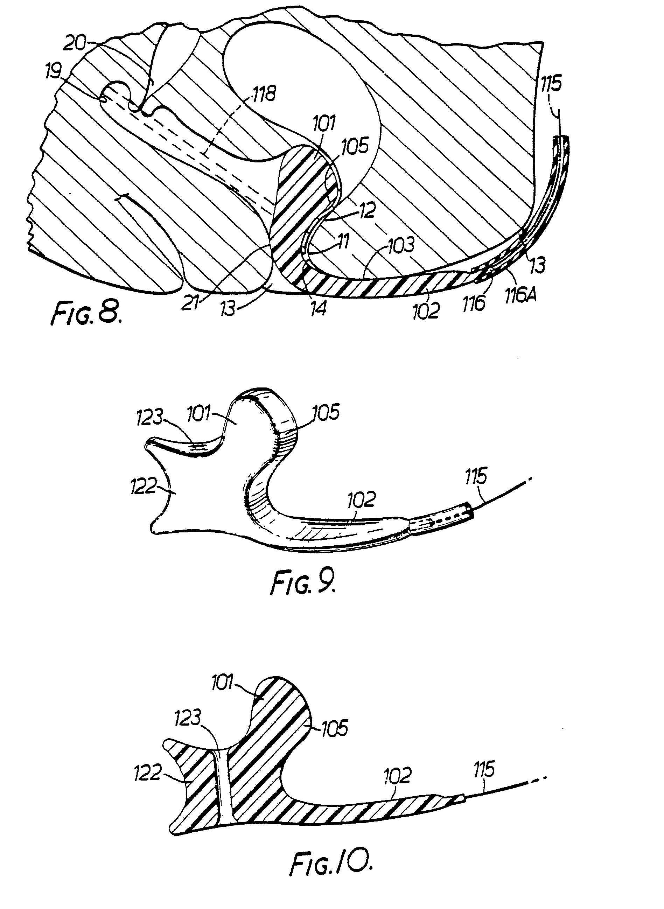

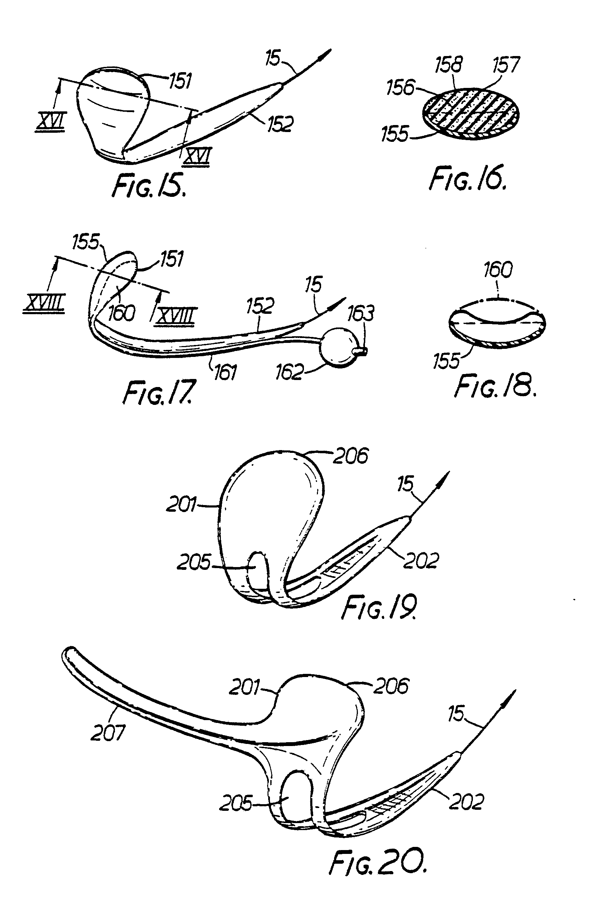

The differences between Figures 3A, 3B and 8 on the one hand and Figure 14 on the other hand reflects the wide range of different shapes and sizes of the vaginal cavity, which in fact really only exists as a cavity when a member is inserted into it. The most constant feature is the location of the anterior dorsal vaginal wall and its relationship to the pubic bone, (25 in Figure 3B). However, even the shape of the pubic bone can vary widely.

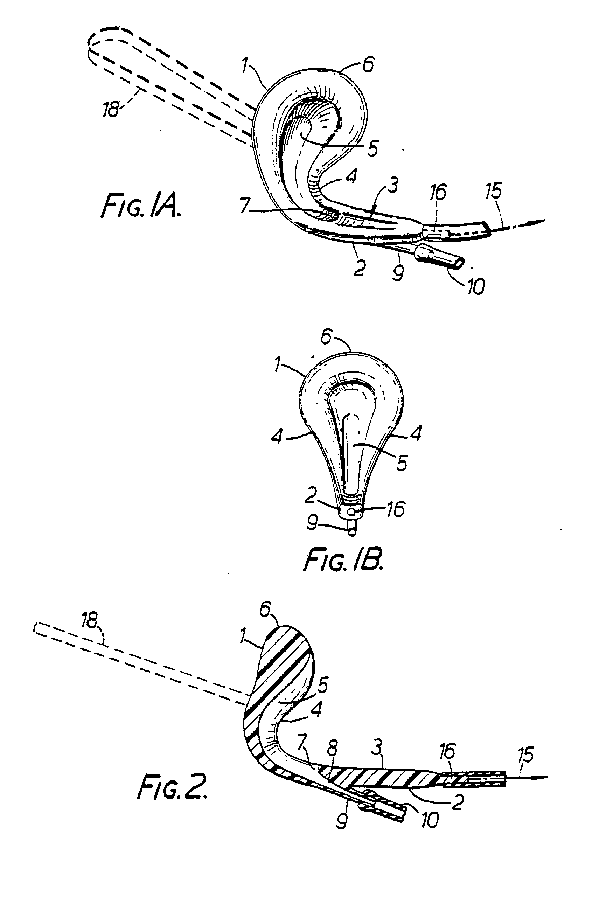

In the first embodiment the invention takes the form shown in Figure 1, and consists of two integrally connected limbs 1 and 2, the inner or upper limb 1 broadening out into a tongue or in elevation (see Figure IB) light bulb shaped plan, and the outer of lower limb 2 being narrower and not broadening out at its end. The limbs 1 and 2 together provide a generally U or V-shaped configuration, the inner face 3 of the lower limb of the U being generally flat or concave. The inner face 4 of the upper limb 1 affords a centrally disposed medially extending duct, channel, or groove 5 defined by the edges of the limb 1, which edges are slightly rounded at its far end 6.

The edges 4 of the channel 5 may also be rounded along its length. The channel issues out of the end 6 of the limb 1 and extends around the base of the U some distance (e.g. about 20 to 30% e.g. 25% of the length) along the lower limb 2, where it terminates on the surface of the limb 2 in a rounded end 7. The channel 5 communicates with an internal duct 8, at or near its end 7, which duct extends more or less diagonally through the limb 2 to emerge through its outer face at 9, at or near the end of the limb 2. This angle enables the bladder to be emptied even when the woman is sitting down. The duct 8 is there connected to, or continues as a tube 10. In use, the limb 1 of the device is inserted into the entrance of the vagina, and is positioned so as to be located in the vagina as shown in Figure 3A, with the limb 1 being placed against the anterior vaginal wall 11 the channel 5 being positioned so as to follow the course of the urethra 12. It is not essential for the limb 1 to extend as far into the vagina as shown in Figure 3A. It may be shorter, provided an adequate seal can be maintained between the vaginal wall 11 and the edges 4 of the channel 5. The lower limb 2 points forwardly and is positioned against the outer surface of the body, within the vulva 13. The urethral opening 14 is thus positioned within the channel 5. Any urine escaping from the urethral opening 14 will pass along the channel 5, through the duct 8 and pass into the tube 10 where it may be collected by any suitable means such as a receptacle attached to the patient's leg.

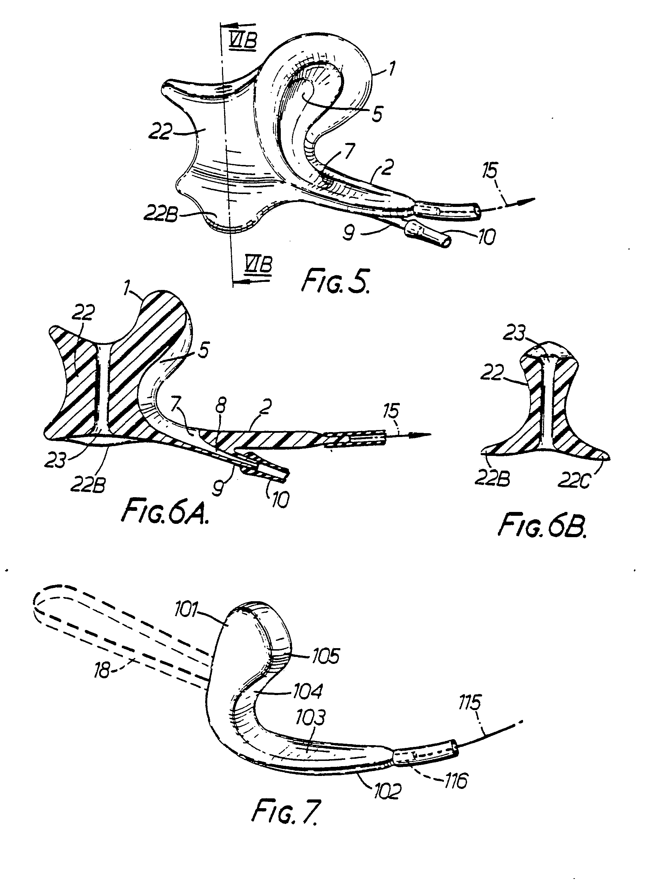

The device is held in position by means of external tensioning means one form of which is a strap 15 which is attached to or is a continuation of the second limb 2 from its forward end 16. The strap 15 (which may be padded or sheathed for comfort) may conveniently be attached to a belt 17 as shown in Figure 4 and is maintained in tension by its own elasticity or that of the belt 17, or both. When the strap 15 is sheathed this is conveniently achieved by a plastics tube 16A which may extend (as shown in Figure 3B) from the end of limb 2 (to which, it may be connected) out beyond the vulva 13, e.g. as far as 16B (see Figure 3B). This prevents the tensioning means rubbing the user. The means 15 may only be elastic within such a tube and thereafter be connected to the belt by an inelastic connection. The belt may be elastic or inelastic. This tension causes the limb 1 to be pressed against the anterior vaginal wall 11. The necessary seal between the vaginal wall 11 and the edges 4 of the channel 5 may be enhanced by lining the edges 4 with any suitable material such as a soft pad, or a cohesive gum of suitable consistency may be used. Suitable gums include a mixture of Karaya gum, glycerine and gelatine mixed to a putty-like consistency, or alternatively liquid polymers such as a cellulose-polybutene combination may be used.

Figure 3B shows a further modification in which a dorsal extension 1A of generally duck bill shape extends rearwardly from the top back face of the limb 1 towards or past the cervix, but stops short of the dorsal wall of the vagina and extends across this region of the vagina. A dorsal extension of this sort is also shown and described in Figure 20 (see reference 207). This extension helps maintain accurate lateral location of the device in use.

In a first modification of the device, the necessary support may be increased internally particularly against lateral movement. This may take the form of a generally rigid but resilient loop 18, which is attached to the back or outer face of the limb 1 as shown in Figures 1 and 3A by dashed lines. In this modification the belt and strap again hold the device in place and cause the limb 1 to be pressed against the anterior vaginal wall 11 in the position previously described. The loop 18 being pressed against the upper dorsal vaginal wall 19 behind the cervix 20 with the natural elasticity of the vaginal wall 19 helps to hold the device in place particularly against lateral movement.

In a second modification of this first embodiment, shown in Figure 5 and in cross section in Figure 6, the device is shaped so as to be held in place between the anterior and dorsal vaginal walls, 11, 21 in the vaginal entrance as well as being held in place by the belt and strap. The limbs I, 2 are generally as described above for the first embodiment, but the base of the U is extended to form a generally saddle-shaped protrusion 22, moulded to conform to the general shape of the lower dorsal vaginal wall 21 and provided on either side and its lower edge by outwardly and downwardly extending wings 22B and 22C which in use bear against the users external body surface outside the vagina. In use, the device is held in position by the combined pressure of the anterior wall 11 on the upper limb 1 and that of the dorsal wall 21 on the saddle-shaped protrusion 22 as well as the belt and strap. The protrusion 22 is provided with a generally vertically disposed duct 23 extending between its upper and lower surfaces, which duct serves to allow egress of natural vaginal fluids, and to permit easy removal of the device.

In a third modification the end of the channel 5 is left open rather than communicating with the duct 8 and tube 10 (which are then no longer needed and can be omitted). This arrangement may thus be similar to the opening 205 described below for Figures 19 and 20.

The device may be made of any suitably flexible material such as rubber, or it may be made of a more rigid material such as polypropylene and provided with pads of compressible material for comfort, in the necessary regions. The structure should be sufficiently rigid to ensure secure location in the vagina and to enable the force exerted by the strap 15 to pull the limb 1 against the vaginal wall 11. If the device is made of a generally rigid material, the base of the U, between the limbs 1 and 2, may be made of a more flexible material to allow some hinging movement between the limbs, thus accommodating personal differences in vaginal shape. When the loop. 18 is present the hinging embodiment can be used or the device can be made of more flexible material throughout. The use of a nonreactive material may be useful to patients with allergies to rubber or silicone products.

This first version, the drainage version, of the invention deals with incontinence by collecting the urine.

The second version, the restrictive version, of the invention mechanically causes retention of the urine.

In the second embodiment, this restrictive version of the invention takes the form shown in Figure 7 and consists of two integrally connected limbs 101, 102. The limbs 101 and 102 together provide a generally U or V-shaped configuration, the inner face 103 of the lower limb 102 being generally flat or concave. The inner face 104 of the upper limb is generally flat or convex and terminates in an inwardly-facing protuberance 105, of which the edges are rounded as shown in Figure 7. In use, the limb 101 of the device is inserted into the entrance to the vagina, and is positioned so as to be located in the vagina as shown in Figure 8 with the limb 101 being placed against the anterior vaginal wall 11, following the line of the urethra 12.

The lower limb 102 points forwardly and is positioned against the outer surface of the body within the vulva 13. The device is held in position by means of tensioning means, one form of which is a strap 115 which is attached to or is a continuation of the lower limb 102 at its forward end 116. The strap 115 (which may be padded for comfort or sheathed as described above in connection with Figure 3B) may conveniently be attached to a belt 17 (as shown in Figure 4 above) and is maintained in tension by its own elasticity or that of the belt 17, or both. This tension causes the limb 101 to be pressed against the anterior vaginal wall 11 directly over the course of the urethra 12. The pressure of the limb 101, and particularly that due to the protuberance 105 causes the urethra 12 to collapse (as shown in Figure 8), so preventing urine flow.

In a first modification of this second embodiment of the device, the necessary support may be increased internally particularly against lateral movement. This may take the form of a rigid loop 118 which is attached to the back or outer face of the limb 101 as shown in Figures 7 and 8 by dashed lines. In this modification, the belt and strap again hold the device in place, the limb 101 being pressed against the anterior vaginal wall 11 in the position previously described. The loop 118 being pressed against the upper dorsal vaginal wall 19 behind the cervix 20 with the natural elasticity of the vaginal wall 19 helps hold the device in place particularly against lateral movement.

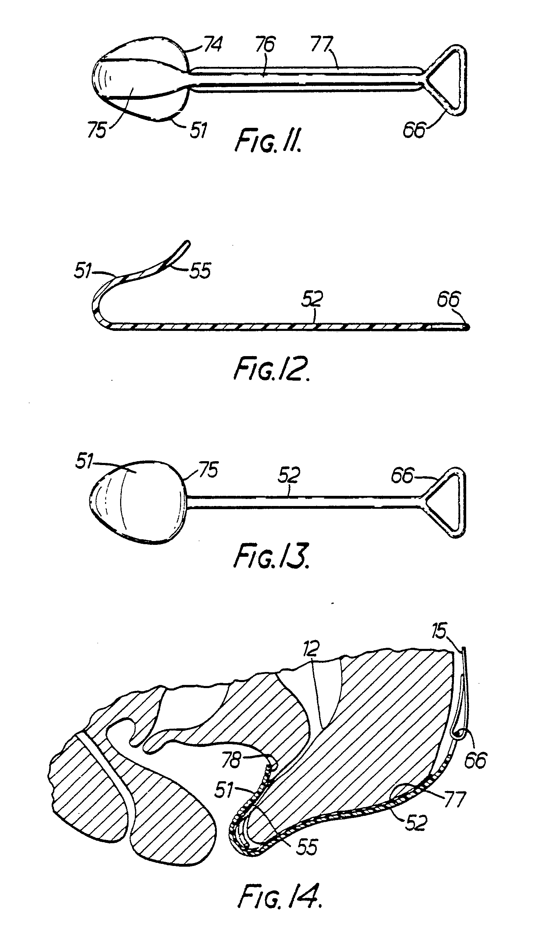

In a second modification of this second embodiment, shown in Figure 9 and in cross section in Figure 10, the device is shaped so as to be held in place between the anterior and dorsal vaginal walls 11, 21, proximate the vaginal entrance as well as being held in place by the belt and strap. The limbs 101 and l02 are generally as described above for the first modification of the second embodiment but the base of the U or V is extended to form a generally saddle-shaped protrusion 122, moulded to conform with the general shape of the lower dorsal vaginal wall 21. In use, the device is held in position by the combined pressure of the anterior wall 11 on the upper limb 1 and that of the dorsal wall 21 on the saddle-shaped protrusion 122 as well as the belt and strap. The protrusion 122 is provided with a generally vertically-disposed duct 123 extending between its upper and lower surfaces, which duct serves to allow egress of natural vaginal fluids and enables the device to be removed easily, and if desired wings 122B and C (not shown) as described above for Figure 5.

It is not essential for the limb 101 to extend as far into the vagina as shown in Figure 8. It may be shorter, provided that it extends far enough above the urethral opening 14 to allow the occlusion of the urethra 12 without significant leakage. The protuberance 105 at the end of the limb 101 is likewise not essential to the invention; the pressure of the limb 101 alone on the urethra 12 can be sufficient to cause it to collapse but the protuberance aids certainty of operation.

The device may be made of any suitably flexible material such as rubber, or it may be made of a more rigid material such as polypropylene, and provided with pads of compressible material, for comfort, in the necessary regions. The structure should be sufficiently rigid to ensure secure location in the vagina, and to enable the force exerted by the strap 15 to pull the limb 101 and the protuberance I05 against the interior vaginal wall 11 sufficiently hard to occlude the urethra 12, If the device is made of a generally rigid material the base of the U, between the limbs 101 and I02, may be made of a more flexible material to allow some hinging movement between the limbs, thus accommodating personal differences in vaginal shape. When the loop 118 is present the hinging embodiment may be used or the device may be made of a more flexible material throughout.

Referring now to Figures 11 to 14 the device in this third modification of the second embodiment is a polypropylene moulding about 3 mms thick. The device before insertion has a shepherd-crook longitudinal cross section having a long straight portion 52 corresponding to the limb 102 having a connecting link 66 at its free end and broadening into a paddle shaped end 75 at the end where it curves round to form a broadened plate 51 of egg saped plan view corresponding to the limb 1 in the first embodiment. This plate 51 is curved inwardly in cross-section towards the portion 52 so as to afford a protuberance 55 effective to apply pressure to the urethra and thus occlude it in use. The straight portion 76 of the limb 52 is preferably sheathed in soft polymer or rubber 77 so as to prevent discomfort or excoriation of the mucous membrane at the lower end and may be made sufficiently flexible to conform in use to the user's body as shown in Figure 14. The edges 74 of the plate 51 are carefully moulded to rounded shapes with the same aim in mind, and in addition the soft sheathing may extend to cover the plate 51 as is also shown at 78 in Figure 14.

Referring now to Figures 15 to 18 these fourth and fifth modifications of the second embodiment are both concerned with restrictive versions of the invention in which the occlusion means can either be overcome by the user (Figures 15 and 16) by internal muscular effort or controlled by external action (Figures 17 and 18).

The arrangement shown in Figures 15 and 16 has an inner or upper limb 151 and an outer or lower limb 152 from the end of which extends tensioning means(indicated diagrammatically at 15) as in the other embodiments. The upper end of the inner limb 152 is oval in cross section (Figure 16) the rigid back face 155 carrying a compressible cushion 157, e.g. having a foam or fluid filled interior 156 and an impervious covering 158. This cushion is of such compressibility as under the pressure of the tensioning means 15 exerted via the back face 155 to occlude the urethra and prevent urine flow but also to be capable of being further compressed when muscular pressure is exerted on the bladder to force urine down the urethra and past this cushion 157.

The arrangement in Figures 17 and 18 has the cushion 157 replaced by a fluid filled bag, e.g. an airbag 160, again mounted on the front side of the rigid back 155 and connected via a tube 161 with a small hand operable pump 162 which in use is located outside the body. In use the pump can be used to inflate the airbag, e.g. to the position shown in dotted lines in Figure 18 to shut off urine flow. The pump 162 is also provided with a release valve 163 is that the airbag can be easily deflated to permit discharge of urine.

In both these modifications, which permit use of the device without removal from the vagina, duct means will be provided to allow discharge of urine. Such a duct may merely be an opening through the device, e.g. the limb 151 or may be a duct such as shown in the arrangement of Figures 1 to 6.

Referring now to Figures 19 and 20 these show the third embodiment of the invention, the stress incontinence version. Here the inner limb 201 is generally of spoon shape whilst the outer limb 202 is narrower. Again the tensioning means extend away from the end of the limb 202 as in the other embodiments and as illustrated diagrammatically at 15. A slot-like duct 205 extends around the bend which joins the limbs 201 and 202 enabling urine to be discharged from the urethral opening 14 without the device needing to be removed from the vagina. The top edge 206 of the inner limb 201 holds the users urethra in or near the normal position and reduces or alleviates stress incontinence.

The arrangement in Figure 20 has a further feature in the dorsal extension 207 which is akin to the extension IA shown in Figure 3B and is of similar duck-bill shape and extent (extending to near the cervix but stopping short thereof and extending across the vagina) and function (helping to maintain lateral location).

In modifications of the arrangements of Figures 19 and 20 the overcomeable or controllable cushion or airbag features of Figures 15 to 18 are incorporated into the structures of the Figure 19 and 20 embodiments.

**Claims**

1. An incontinence device for use by females, the device being provided with an internal limb (1, 101, 51, 151, 201) generally shaped so as, in use, to be located in the vagina and to conform to or contact the anterior vaginal wall (11) of the user; an external portion (2, 102, 52, 152, 202) arranged, in use, to extend out of the vaginal opening; and tensioning means (15, 115) external to the user in use of the device, attached to the external portion, the tensioning means being adapted to be secured to or mounted on the user's body externally, or to or on the user's clothing, whereby the internal limb is urged into or held in functional cooperation with the urethra (12), the internal limb and the external portion together forming a generally U- or V-shaped configuration, characterised in that the external portion consists of a lower external limb (2, 102, 52, 152, 202) which is generally shaped such that, in use, the tensioning means (15, 115) pulls the lower limb forwards so that the lower limb is positioned at least partially within the vulva (13) and against the pubic bone (25).

2. An incontinence device as claimed in claim 1, which is arranged, in use, so that the internal limb (1) fits around the opening (14) to the urethra (12) and the device is provided with duct means (8,I0) to permit discharge of urine from the urethra through the device.

3. An incontinence device as claimed in claim 1, in which the internal limb (101, 51, 151) is provided with occlusion means (105, 55, 157, 160) arranged in use to occlude the urethra (12) and thus prevent discharge of urine from the bladder.

4. A device as claimed in claim 3 in which the occluion means comprise a protuberance (105, 55, 157, 160) which presses against the urethra (12).

5. A device as claimed in claim 4 in which the protuberance (160) can be increased in size or reduced in size by control means located externally in use.

6. An incontinence device as claimed in Claim 1, in which the internal limb (201) is arranged in use to hold the users urethra (12) in a normal or approximately normal position whereby stress incontinence is substantially alleviated or eliminated.

7. An incontinence device as claimed in claim 1, which is arranged, in use, so that the internal limb (151) fits around the opening (14) to the urethra 12 and the device is provided with duct means to permit discharge of urine from the urethra thorugh the device, the internal limb also being provided with occlusion means (160) arranged in use to controllably or overcomably occlude the urethra so as to permit the user at will to permit or cause discharge of urine from the bladder.

8. An incontinence device as claimed in any one of the preceding claims in which the device is held in position by a rearwardly extending portion (22, 122) which is pressed against the dorsal vaginal wall (21).

9. An incontinence device as claimed in claim 8 in which the rearwardly extending portion is generally shaped so as to conform to the lower dorsal vaginal wall proximate to the vaginal opening.

10. An incontinence device as claimed in any one of the preceding claims in which a rearwardly extending portion (1A, 18, 118) extends from the region of the upper end of the internal limb and across the vagina laterally and extends into the region of the cervix (20) but stops short thereof.

11. An incontinence device as claimed in any one of the preceding claims in which the tensioning means comprises a strap (15, 115) or cord attached at one end to a belt (17), the tension being due to the elasticity of the cord or that of the belt, or both.

12. An incontinence device as claimed in any one of the preceding claims in which the external tensioning means provides the sole external means of securing the device in position and of urging or holding the internal limb (1, 101, 51, 151, 201) in functional cooperation with the urethra (12).

**Patentansprüche**

1. Inkontinenzeinrichtung für die Benutzung durch weibliche Personen mit einem inneren Schenkel (1, 101, 51, 151, 201), der allgemein so geformt ist, daß er sich im Gebrauch der Einrichtung in der Scheide befindet und der vorderen Scheidenwand (11) der Trägerin angepaßt ist oder diese Wand berührt; mit einem äußeren Teil (2, 102, 52, 152, 202), der so angeordnet ist, daß er sich im Gebrauch der Einrichtung aus der Scheidenmündung heraus erstreckt; und mit einer Spanneinrichtung (15, 115), die sich im Gebrauch der Einrichtung außerhalb des Körpers der Trägerin befindet und die an dem Körper oder der Kleidung der Trägerin außen derart befestigbar oder anbringbar ist, daß der innere Schenkel im funktionellen Zusammenwirken mit der Harnröhre gehalten oder zum funktionellen Zusammenwirken mit der Harnröhre (12) veranlaßt wird, wobei der innere Schenkel zusammen mit dem äußeren Teil eine im wesentlichen U- oder V-förmige Konfiguration bilden, dadurch gekennzeichnet, daß der äußere Teil aus einem unteren, äußeren Schenkel (2, 102, 52, 152, 202) besteht, der allgemein so geformt ist, daß im Gebrauch der einrichtung die Spanneinrichtung (15, 115) den unteren Schenkel derart vorwärtszieht, daß sich der untere Schenkel mindestens teilweise in der Vulva (13) befindet und an das Schambein (25) angehalten wird.

2. Inkontinenzeinrichtung nach Anspruch 1, die im Gebrauch so angeordnet ist, daß sich der innere Schenkel (1) um die Mündung (14) zur Harnröhre (12) herum erstreckt, und daß die Einrichtung mit einer Kanalanordnung (8, 10) versehen ist, durch die hindurch Harn aus der Harnröhre durch die Einrichtung treten kann.

3. Inkontinenzeinrichtung nach Anspruch 1, dadurch gekennzeichnet, daß der innere Schenkel (101, 51, 151) mit einer Absperreinrichtung (105, 55, 157, 160) versehen ist, die so angeordnet ist, daß sie im Gebrauch der Einrichtung die Harnröhre (12) absperrt, um einen Austritt von Harn aus der Blase zu verhindern.

4. Einrichtung nach Anspruch 3, dadurch gekennzeichnet, daß die Absperreinrichtung eine gegen die Harnröhre (12) drückende Erhöhung (105, 55, 157, 160) besitzt.

5. Einrichtung nach Anspruch 4, dadurch gekennzeichnet, daß die Erhöhung (160) durch eine im Gebrauch der Einrichtung extern angeordnete Steuereinrichtung vergrößerbar oder verkleinerbar ist.

6. Inkontinenzeinrichtung nach Anspruch 1, dadurch gekennzeichnet, daß der innere Schenkel (201) im Gebrauch der Einrichtung so angeordnet ist, daß er die Harnröhre (12) der Trägerin in einer normalen oder annähernd normalen Stellung hält, so daß eine Spannungsinkontinenz im wesentlichen vermindert oder vermieden wird.

7. Inkontinenzeinrichtung nach Anspruch 1, die im Gebrauch so angeordnet ist, daß sich der innere Schenkel (151) um die Mündung (14) zur Harnröhre (12) herum erstreckt, daß die Einrichtung mit einer Kanalanordnung Versehen ist, in der Harn aus der Harnröhre durch die Einrichtung treten kann, und daß der innere Schenkel mit einer Absperreinrichtung (160) versehen ist, die so angeordnet ist, daß sie im Gebrauch der Einrichtung eine steuerbare oder überwindbare Absperrung der Harnröhre bewirken kann, so daß die Trägerin willkürlich einen Austritt von Harn aus der Blase zulassen oder herbeiführen kann.

8. Inkontinenzeinrichtung nach einem der vorhergehenden Ansprüche, dadurch gekennzeichnet, daß die Einrichtung von einem rückwärtsgerichteten und gegen die dorsale Scheidenwand (21) gedrückten Teil (22, 122) in Stellung gehalten wird.

9. Inkontinenzeinrichtung nach Anspruch 8, dadurch gekennzeichnet, daß der rückwärtsgerichtete Teil allgemein so geformt ist, daß er sich in der Nähe der Scheidenmündung dem unteren Teil der dorsalen Scheidenwand anpaßt.

10. Inkontinenzeinrichtung nach einem der vorhergehenden Ansprüche, dadurch gekennzeichnet, daß sich von dem oberen Endbereich des inneren Schenkels ein rückwärts gerichteter Teil (1A, 18, 118) quer durch die Scheide erstreckt und bis in den Bereich des Gebärmutterhalses (20) reicht, aber knapp vor ihm endet.

11. Inkontinenzeinrichtung nach einem der vorhergehenden Ansprüche, dadurch gekennzeichnet, daß die Spanneinrichtung ein Band (15, 115) oder eine Schnur umfaßt, das bzw. die am einen Ende an einem Gurt (17) angebracht ist, und daß die Spannung durch die Elastizität der Schnur und/ oder des Gurtes bedingt ist.

12. Inkontinenzeinrichtung nach einem der vorhergehenden Ansprüche, dadurch gekennzeichnet, daß die außen angeordnete Spanneinrichtung die einzige außen angeordnete Einrichtung ist, die dazu dient, die Einrichtung in Stellung zu halten und den inneren Schenkel (1, 101, 51, 151, 201) zum funktionellen Zusammenwirken mit der Harnröhre (12) zu veranlassen oder im funktionellen Zusammenwirken mit der Harnröhre (12) zu halten.

**Revendications**

1. Dispositif d'incontinence destiné à être utilisé par des femmes, comportant une aile interne (1, 101, 51, 151, 201) façonnée dans son ensemble de façon à être disposée, en service, dans le vagin et à épouser la paroi vaginale antérieure (11) de l'utilisatrice ou à être en contact avec celle-ci; une partie externe (2, 102, 52, 152, 202) disposée, en service, de manière à s'étendre hors de l'orifice vaginal; et un moyen tendeur (15, 115) extérieur à l'utilisatrice lorsque le dispositif est en service, attaché à la partie externe, le moyen tendeur pouvant être fixé extérieurement au corps de l'utilisatrice ou à ses vêtements ou pouvant y être monté, de sorte que l'aile interne est sollicitée ou maintenue en contact de coopération fonctionnelle avec l'urètre (12), l'aile interne et la partie externe formant ensemble une configuration en substance en U ou en V, caractérisé en ce que la partie externe comprend une aile externe inférieure (2, 102, 52, 152, 202) qui est dans l'ensemble façonnée de telle manière, qu'en service, le moyen tendeur (15, 115) tire l'aile inférieure vers l'avant de telle sorte qu'elle soit positionée au moins partiellement dans la vulve (13) et contre l'os pubien (25).

2. Dispositif d'incontinence suivant la revendication 1 qui est agencé, en service, de telle façon que l'aile interne (1) s'ajuste autour de l'orifice (14) de l'urètre (12) et qui est pourvu de conduits (8, 10) permettant l'évacuation de l'urine de l'urètre à travers le dispositif.

3. Dispositif d'incontinence suivant la revendication 1, dans lequel l'aile interne (101, 51, 151) est pourvu d'un moyen d'occlusion (105, 55, 157, 160) servant à occlure l'urètre (12) et à empêcher ainsi l'évacuation de l'urine de la vessie.

4. Dispositif suivant la revendication 3, dans lequel le moyen d'occlusion comprend une protubérance (105, 55, 157, 160) qui exerce une pression contre l'urètre (12).

5. Dispositif suivant la revendication 4, dans lequel le volume de la protubérance (160) peut être augmenté ou réduit par un dispositif de commande disposé à l'extérieur, en service.

6. Dispositif d'incontinence suivant la revendication 1, dans lequel l'aile interne (201) sert à retenir l'urètre (12) de l'utilisatrice dans une position normale ou approximativement normale, de telle sorte que l'incontinence de stress soit en substance atténuée ou éliminée.

7. Dispositif d'incontinence suivant la revendication 1, qui est agencé, en service, de telle façon que l'aile interne (151) s'ajuste autour de l'orifice (14) de l'urètre (12) et qui comprend un conduit pour permettre l'évacuation d'urine de l'urètre à travers le dispositif, l'aile interne étant également pourvue d'un moyen d'occlusion (160) servant à occlure l'urètre d'une manière pouvant être commandée ou surmontée, afin de conférer à l'utilisatrice la possibilité de permettre ou de provoquer, à volonté, l'évacuation d'urine de la vessie.

8. Dispositif d'incontinence suivant l'une quelconque des revendications précédentes, qui est retenu en position par une partie s'étendant vers l'arrière (22, 122) qui est pressée contre la paroi vaginale dorsale (21).

9. Dispositif d'incontinence suivant la revendication 8, dans lequel la partie s'étendant vers l'arrière est dans l'ensemble façonnée de manière à épouser la paroi vaginale dorsale inférieure à proximité de l'orifice du vagin.

10. Dispositif d'incontinence suivant l'une quelconque des revendications précédentes, dans lequel une partie s'étendant vers l'arrière (1A, 18, 118) part de la région de l'extrémité supérieure de l'aile interne et s'étend en travers du vagin latéralement et dans la région du col de l'utérus (20), mais s'arrête à courte distance de celui-ci.

11. Dispositif d'incontinence suivant l'une quelconque des revendications précédentes, dans lequel le moyen tendeur comprend une sangle (15, 115) ou un cordon attaché par une extrémité à une ceinture (17), la tension étant due à l'élasticité du cordon et/ou à celle de la ceinture.

12. Dispositif d'incontinence suivant l'une quelconque des revendications précédentes, dans lequel le moyen tendeur externe constitue le seul moyen externe pour fixer le dispositif en place et pour solliciter ou retenir l'aile interne (1, 101, 51, 151, 201) en contact de coopération fonctionnelle avec l'urètre (12).

FIG. IA.

FIG. IB.

FIG. 2.

FIG.3A.

FIG.3B.

FIG.4.

FIG. 5.

FIG. 6A.

FIG. 6B.

FIG. 7.

FIG. 8.

FIG. 9.

FIG. 10.

FIG. II.

FIG. 12.

FIG. 13.

FIG. 14.

*Fig. 15.*

*Fig. 16.*

*Fig. 17.*

*Fig. 18.*

*Fig. 19.*

*Fig. 20.*

6